# EUROPÄISCHE PATENTANMELDUNG

(11) **EP 1 508 347 A1**
(43) Veröffentlichungstag der Anmeldung: **23.02.2005**
(21) Anmeldenummer: 04016624.1
(22) Anmeldetag: 15.07.2004
(51) Int. Cl.: A61M 16/06

(54) **Beatmungsmaske mit Kopfhaube**

(30) Priorität: 20.08.2003 DE 10338169
(71) Anmelder: Weinmann Geräte für Medizin GmbH & Co. KG, 22525 Hamburg (DE)
(72) Erfinder: Schumacher, Gerhard, 32267 Bünde (DE)
(74) Vertreter: Klickow, Hans Henning,Dr.-Ing.

(57) **Zusammenfassung**

Die Beatmungsmaske (1) ist mit einer Kopfhaube (2) verbunden, die mindestens ein Stirnband (5) sowie mindestens ein Nackenband (6) aufweist. Das Stirnband (5) und das Nackenband (6) sind im Bereich ihrer der Beatmungsmaske (1) abgewandten Ausdehnungen miteinander verbunden. Das Stirnband (5) ist in einer Benutzungsorientierung oben und das Nackenband (6) seitlich an der Beatmungsmaske (1) befestigt. Benachbart zur Beatmungsmaske (1) angeordnete Bereiche des Nackenbandes (6) sind durch ein Kinnband (13) miteinander verbunden. Das Kinnband (13) erstreckt sich im wesentlichen in einer Längsrichtung des Nackenbandes (6). Endsegmente (7,8) des Nackenbandes (6), die zwischen den Kopplungsstellen des Nackenbandes (6) und des Kinnbandes (13) sowie der Beatmungsmaske (1) angeordnet sind, verlaufen in Richtung auf die Beamtungsmaske (1) relativ zum Kinnband (13) geneigt und mit zunehmendem Abstand zum Kinnband (13).

## Beschreibung

Die Erfindung betrifft eine Beatmungsmaske mit Kopfhaube, wobei die Kopfhaube mindestens ein Stirnband sowie mindestens ein Nackenband aufweist, die im Bereich ihrer der Beatmungsmaske abgewandten Ausdehnungen miteinander verbunden sind, sowie bei der das Stirnband in einer Benutzungsorientierung oben und das Nackenband seitlich an der Beatmungsmaske befestigt sind und bei der benachbart zur Beatmungsmaske angeordnete Bereiche des Nackenbandes durch ein Kinnband miteinander verbunden sind.

Derartige Kopfhauben für Beatmungsmasken sind bereits in unterschiedlichen Ausführungsformen bekannt. Die Nackenbänder der bekannten Kopfmasken sind typischerweise derart konstruiert, daß sie bei einer Benutzung im wangenbereich unterhalb der Ohren vorbeilaufen. Bekannt sind ebenfalls Kopfhauben mit zusätzlichen seitlichen Kopfriemen, die ebenfalls dicht oberhalb der Ohren des Patienten verlaufen. Die funktionelle Auslegung der bekannten Kopfmasken erfolgt im wesentlichen derart, daß eine Optimierung hinsichtlich der Befestigung der Maske auf dem Gesicht des Patienten erfolgt. Ausgehend von dieser konstruktiven Vorgabe ergeben sich auftretende Zugwinkel sowie Zugkräfte im Bereich des Nackenbandes sowie des Kopfbandes eher zufällig und in der Regel ungleichmäßig aufgeteilt.

Zur Verhinderung einer Mundatmung während einer mit Hilfe der Beatmungsmaske durchgeführten Beatmung ist es ebenfalls bereits bekannt, zusätzlich ein Kinnband zu verwenden, das eine ungewollte Öffnung des Mundes während eines Schlafes des Patienten verhindert. Das Kinnband ist üblicher Weise als eine Kinnbinde ausgebildet.

Bei einer Verwendung der bekannten Kopfhauben empfindet es der Patient häufig als unangenehm, daß durch ungünstige Zugwinkel sowie ungleiche Krafteinleitungen lokal unangenehme Krafteinleitungen in den Kopfbereich erfolgen und daß durch den Verlauf der Bänder im Bereich der Ohren Scheuerstellen an den Ohren auftreten.

Aufgabe der vorliegenden Erfindung ist es, eine Beatmungsmaske mit Kopfhaube der einleitend genannten Art derart zu konstruieren, daß sowohl eine funktionelle Befestigung der Beatmungsmaske im Gesicht des Patienten unterstützt als auch ein angenehmer Tragekomfort erreicht wird.

Diese Aufgabe wird erfindungsgemäß dadurch gelöst, daß sich das Kinnband im wesentlichen in einer Längsrichtung des Nackenbandes erstreckt und das Endsegmente des Nackenbandes, die zwischen den Kopplungsstellen des Nackenbandes mit dem Kindband sowie der Beatmungsmaske angeordnet sind, in Richtung auf die Beatmungsmaske relativ zum Kinnband geneigt und mit zunehmenden Abstand zum Kinnband verlaufen.

Durch die relativ zum Verlauf des Kinnbandes geneigten Anordnung der Endsegmente des Nackenbandes wird ein Verlauf des Nackenbandes unterstützt, der sowohl eine sichere Fixierung der Beatmungsmaske im Bereich des Gesichtes des Patienten gewährleistet als auch Scheuerstellen im Bereich der Ohren des Patienten vermeidet. Darüber hinaus wird eine gleichmäßige Verteilung der Zugwinkel sowie eine gleichmäßige Verteilung der zugkräfte unterstützt. Im Ergebnis wird eine einheitlich konstruierte Kopfhaube mit Kinnbänderung bereitgestellt.

Durch die geneigte Anordnung der Endsegmente des Nackenbandes werden sowohl in lotrechter Richtung nach unten als auch bezüglich des Kopfes des Patienten nach hinten gerichtete Kräfte in die Beatmungsmaske eingeleitet. Da gleichzeitig über das Stirnband nach oben sowie bezüglich des Kopfes des Patienten nach hinten gerichtete Kräfte in die Beatmungsmaske eingeleitet werden, wird eine allseitig exakt definierte Positionierung der Atemmaske im Gesicht des Patienten unterstützt und diese Positionierung wird auch bei Belastungen, die durch Bewegungen des Patienten oder Bewegungen des Beatmungsschlauches in die Maske eingeleitet werden, mit hoher Zuverlässigkeit gehalten.

Eine vorteilhafte Dimensionierung zur Gewährleistung sowohl einer hohen Fixiersicherheit für die Beatmungsmaske als auch zur Erreichung eines hohen Benutzungskomforts wird dadurch definiert, daß ein Neigungswinkel zwischen dem Kinnband und mindestens einem der Endsegmente einen Wert im Bereich von 10 Grad bis 70 Grad aufweist.

Als besonders zweckmäßig hat es sich erwiesen, daß der Neigungswinkel einen Wert von etwa 20 Grad bis 45 Grad aufweist.

Eine kompakte Gestaltung wird dadurch unterstützt, daß die Kopfhaube genau ein Stirnband aufweist.

Eine örtlich verteilte Krafteinleitung kann dadurch erreicht werden, daß die Kopfhaube genau zwei Stirnbänder aufweist.

Ein typisches Anwendungsgebiet wird dadurch definiert, daß eine konstruktive Gestaltung der Kopfhaube zur Unterstützung einer CPAP-Beatmung vorliegt.

Zur weiteren Erhöhung des Benutzungskomforts ist daran gedacht, daß das Stirnband aus einem flexiblen Material ausgebildet ist.

Ebenfalls trägt es zu einem hohen Benutzungskomfort bei, daß das Nackenband aus einem flexiblen Material ausgebildet ist.

Eine vorteilhafte Zugwinkelorientierung wird dadurch unterstützt, daß das Stirnband im Bereich seiner Einmündung in das Nackenband etwa rechtwinklig zum Nackenband verläuft.

Zur Unterstützung einer einfachen Fertigung wird vorgeschlagen, daß die Endsegmente einteilig am Nackenband angeformt sind.

Eine einfache Geometrie der einzelnen Bauelemente wird dadurch erreicht, daß die Endsegmente separat vom Nackenband gefertigt und am Nackenband fixiert sind.

zur weiteren Erhöhung des Benutzungskomforts ist vorgesehen, daß im Bereich eines mit einem Grundkörper der Beatmungsmaske verbundenen oberen Befestigungselementes eine Stirnstütze angeordnet ist.

Eine Anpassung an die Anatomie unterschiedlicher Patienten wird dadurch erleichtert, daß die Stirnstütze positionierbar im Bereich des oberen Befestigungselementes angeordnet ist.

Eine Abstandsvorgabe zwischen der Beatmungsmaske und dem Kinnband wird dadurch unterstützt, daß im Bereich mindestens eines der Endsegmente eine Längenjustierung angeordnet ist.

Ein verbesserter Einstellungskomfort kann dadurch erreicht werden, daß im Bereich des Nackenbandes mindestens eine Längenjustierung angeordnet ist.

Eine Längenanpassung im Bereich des Nackenbandes wird dadurch bereitgestellt, daß im Bereich des Nackenbandes mindestens ein Verschluß angeordnet ist.

Eine Verwendung sowohl durch Rechtshänder als auch durch Linkshänder wird dadurch unterstützt, daß das Nackenband zwei jeweils seitlich und einander gegenüberliegend angeordnete Verschlüsse aufweist.

In den Zeichnungen sind Ausführungsbeispiele der Erfindung schematisch dargestellt. Es zeigen:
- Fig. 1: eine perspektivische Darstellung einer im Bereich des Kopfes des Patienten angeordneten Beatmungsmaske mit Kopfhaube,
- Fig. 2: eine Darstellung der Beatmungsmaske mit Kopfhaube gemäß Blickrichtung II in Fig. 1 und
- Fig. 3: eine Darstellung der Beatmungsmaske mit Kopfhaube gemäß Blickrichtung III in Fig. 2.

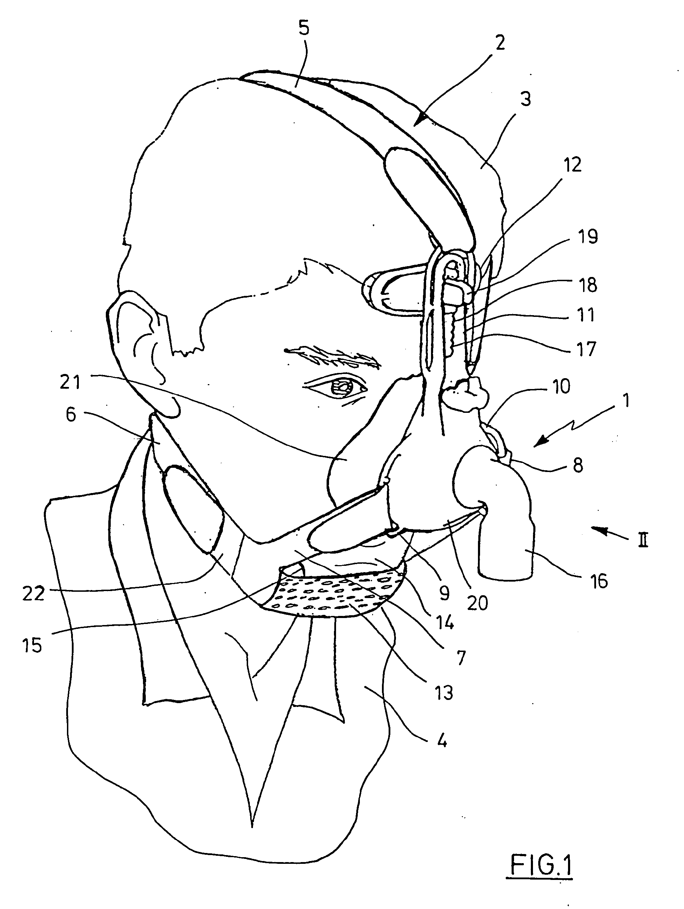

Die Darstellung in Fig. 1 veranschaulicht die Anordnung einer Beatmungsmaske (1) mit Kopfhaube (2) im Bereich des Kopfes (3) eines Patienten (4). Die Kopfhaube (2) weist ein Stirnband (5) sowie ein Nackenband (6) auf. Das Nackenband (6) ist über Endsegmente (7, 8) mit seitlichen Befestigungselementen (9, 10) der Beatmungsmaske (1) verbunden.

Das Stirnband (5) ist über ein oberes Befestigungselement mit der Kopfhaube (2) verbunden. Im Bereich des oberen Befestigungselementes (11) ist darüber hinaus eine Stirnstütze (12) positionierbar geführt. In einem in Fig. 1 nicht erkennbaren Bereich hinter dem Kopf (3) sind das Stirnband (5) und das Nackenband (6) miteinander verbunden.

Im Bereich von Übergängen der Endsegmente (7, 8) in den weiteren Bereich des Nackenbandes (6) ist ein Kinnband (13) befestigt, das bei dem in Fig. 1 veranschaulichten Benutzungszustand über ein Kinn (14) des Patienten (4) verläuft. In Richtung auf die Beatmungsmaske (1) verlaufen die Endsegmente (7, 8) unter einem Neigungswinkel (15) mit einem zunehmenden Abstand zum Kinnband (13).

Im Bereich der Beatmungsmaske (1) ist ein Kupplungselement (16) zum Anschluß eines Beatmungsschlauches angeordnet.

Insbesondere ist daran gedacht, das Kupplungselement (16) beweglich im Bereich der Beatmungsmaske (1) anzuordnen. Die Beweglichkeit kann beispielsweise durch eine kugelgelenkartige Realisierung bereitgestellt werden. Hinsichtlich der Positionierbarkeit der Stirnstütze (12) ist daran gedacht, diese sowohl in vertikaler Richtung entlang einer Stirn des Patienten (4) als auch quer zur Stirn positionierbar anzuordnen. Die Positionierbarkeit der Stirnstütze (12) kann beispielsweise durch einen mit Rastungen (17) versehenen Schlitz (18) im Bereich des oberen Befestigungselementes (11) realisiert werden, innerhalb dessen die Stirnstütze (12) mit einem Sockelelement (19) festgeklemmt wird. Als Material für das Stirnband (5) und das Nackenband (6) kann beispielsweise ein flexibles Gewebe verwendet werden. Als Kinnband (13) wird ein weiches feuchtigkeitsdurchlässiges Gewebe eingesetzt. Ein Grundkörper (20) der Beatmungsmaske (1) besteht vorzugsweise aus einem mechanisch stabilen Kunststoff, ein Konturelement (21) der Beatmungsmaske (1), das zur Anlage am Gesicht des Patienten (4) vorgesehen ist, besteht aus einem sehr weichen und nachgiebigen Kunststoff.

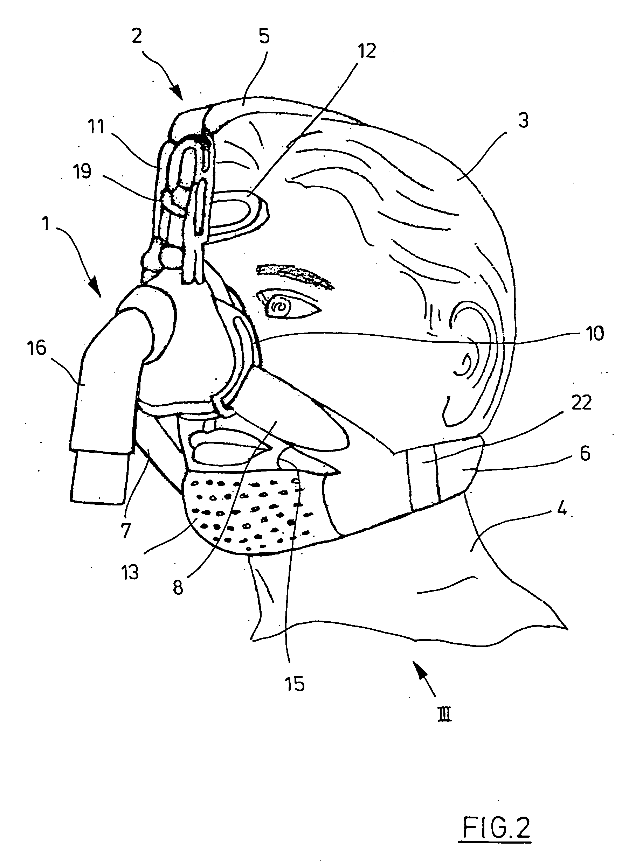

Fig. 2 veranschaulicht die Anordnung der Beatmungsmaske (1) mit Kopfhaube (2) in einer anderen perspektivischen Darstellung. Insbesondere ist noch einmal die Anordnung des Neigungswinkels (15) sowie die hieraus resultierende geneigte Anordnung der Endsegmente (7, 8) relativ zum Kinnband (13) veranschaulicht.

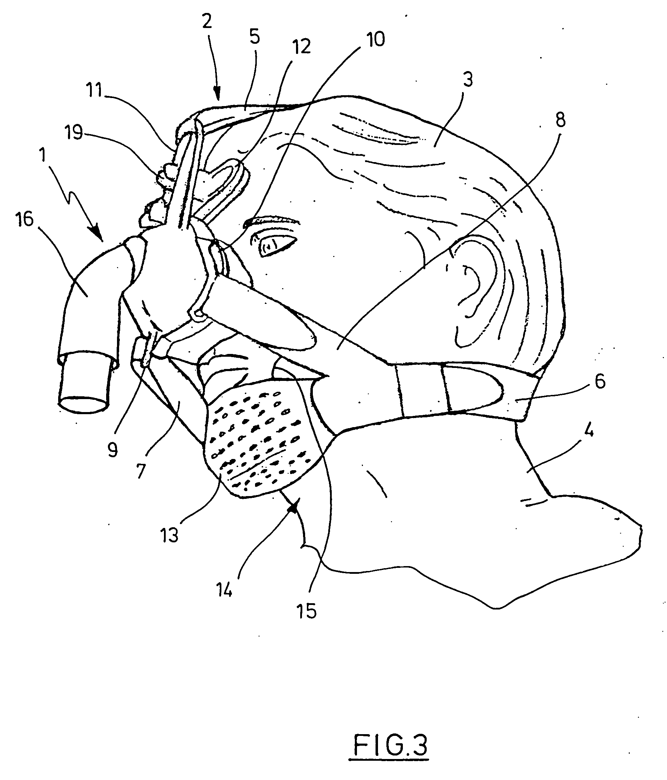

Aus der Darstellung in Fig. 3 ist zu erkennen, daß sich das Kinnband (13) großflächig um das Kinn (14) des Patienten (4) herumerstreckt. Hierdurch wird im Bereich des Kopfes des Patienten (4) eine gute und gleichmäßige Aufnahme sowohl durch das Nackenband (6) eingeleiteter nach hinten wirkender Kräfte als auch über das Stirnband (5) und die Beatmungsmaske (1) sowie die Endsegmente (7, 8) eingeleiteter nach oben gerichteter Kräfte unterstützt. Gleichzeitig wird hierdurch eine Fixierung des Kinns (14) des Patienten (4) bereitgestellt, die ein ungewolltes Öffnen des Mundes verhindern.

Aus den Figuren ist insbesondere auch zu erkennen, daß sowohl die Endsegmente (7, 8) als auch das Nackenband (6) mit einer Längenjustierung versehen sind. Bei den dargestellten Ausführungsbeispielen ist die Längenjustierung schlaufenartig ausgeführt und die einander zugewandten Flächen der Bauelemente können mit Klettverschlüssen versehen sein. Nach einem Lösen der Klettverschlüsse können beispielsweise die Enden der Endsegmente (7, 8) geeignet durch die seitlichen Befestigungselemente (9, 10) verschoben werden und eine jeweilige Längenanpassung vorgenommen werden. Hinsichtlich des Nackenbandes (6) ist es möglich, entweder lediglich eine Längenjustierung vorzusehen oder eine beidseitige Anordnung von zwei Längenjustierungen zu realisieren, um eine Handhabung sowohl durch Linkshänder als auch durch Rechtshänder zu unterstützen.

Darüber hinaus ist bei den dargestellten Ausführungsbeispielen das Nackenband (6) mit zwei Verschlüssen (22) versehen, die als Schnellverschlüsse ausgeführt sind. Grundsätzlich ist auch hier die Verbindung eines Verschlusses (22) ausreichend, die Verwendung von zwei Verschlüssen (22) unterstützt aber auch in diesem Fall eine Verwendung sowohl durch Linkshänder als auch durch Rechtshänder. Die Verschlüsse (22) ermöglichen es, ein Anliegen und Abnehmen der Beatmungsmaske (1) ohne eine Längenveränderung des Nackenbandes (6) zu realisieren. Nach einem Abnehmen und einem erneuten Anlegen der Beatmungsmaske (1) sind somit die ursprünglichen Längeneinstellungen wieder unverändert verfügbar.

## Patentansprüche

1. Beatmungsmaske mit Kopfhaube, wobei die Kopfhaube mindestens ein Stirnband sowie mindestens ein Nackenband aufweist, die im Bereich ihrer der Beatmungsmaske abgewandten Ausdehnungen miteinander verbunden sind, so wie bei der das Stirnband in einer Benutzungsorientierung oben und das Nackenband seitlich an der Beatmungsmaske befestigt sind und bei der benachbart zur Beatmungsmaske angeordnete Bereiche des Nackenbandes durch ein Kinnband miteinander verbunden sind, **dadurch gekennzeichnet, daß** sich das Kinnband (13) im wesentlichen in einer Längsrichtung des Nackenbandes (6) erstreckt und das Endsegmente (7, 8) des Nackenbandes (6), die zwischen den Kopplungsstellen des Nackenbandes (6) und dem Kinnband (13) sowie der Beatmungsmaske (1) angeordnet sind, in Richtung auf die Beatmungsmaske (1) relativ zum Kinnband (13) geneigt und mit zunehmendem Abstand zum Kinnband (13) verlaufen.

2. Beatmungsmaske nach Anspruch 1, **dadurch gekennzeichnet, daß** ein Neigungswinkel (15) zwischen dem Kinnband (13) und mindestens einem der Endsegmente (7, 8) einen Wert im Bereich von 10 Grad bis 70 Grad aufweist.

3. Beatmungsmaske nach Anspruch 1 oder 2, **dadurch gekennzeichnet, daß** der Neigungswinkel (15) einen Wert von etwa 20 Grad bis 45 Grad aufweist.

4. Beatmungsmaske nach einem der Ansprüche 1 bis 3, **dadurch gekennzeichnet, daß** die Kopfhaube (2) genau ein Stirnband (5) aufweist.

5. Beatmungsmaske nach einem der Ansprüche 1 bis 3, **dadurch gekennzeichnet, daß** die Kopfhaube (2) genau zwei Stirnbänder (5) aufweist.

6. Beatmungsmaske nach einem der Ansprüche 1 bis 5, **dadurch gekennzeichnet, daß** eine konstruktive Gestaltung der Kopfhaube (2) zur Unterstützung einer CPAP-Beatmung vorliegt.

7. Beatmungsmaske nach einem der Ansprüche 1 bis 6, **dadurch gekennzeichnet, daß** das Stirnband (5) aus einem flexiblen Material ausgebildet ist.

8. Beatmungsmaske nach einem der Ansprüche 1 bis 6, **dadurch gekennzeichnet, daß** das Nackenband (6) aus einem flexiblen Material ausgebildet ist.

9. Beatmungsmaske nach einem der Ansprüche 1 bis 8, **dadurch gekennzeichnet, daß** das Stirnband (5) im Bereich seiner Einmündung in das Nackenband (6) etwa rechtwinklig zum Nackenband (6) verläuft.

10. Beatmungsmaske nach einem der Ansprüche 1 bis 9, **dadurch gekennzeichnet, daß** die Endsegmente (7, 8) einteilig am Nackenband (6) angeformt sind.

11. Beatmungsmaske nach einem der Ansprüche 1 bis 9, **dadurch gekennzeichnet, daß** die Endsegmente (7) separat vom Nackenband (6) gefertigt und am Nackenband (6) fixiert sind.

12. Beatmungsmaske nach einem der Ansprüche 1 bis 11, **dadurch gekennzeichnet, daß** im Bereich eines mit einem Grundkörper (20) der Beatmungsmaske (1) verbundenen oberen Befestigungselementes (11) eine Stirnstütze (12) angeordnet ist.

13. Beatmungsmaske nach Anspruch 12, **dadurch gekennzeichnet, daß** die Stirnstütze (12) positionierbar im Bereich des oberen Befestigungselementes (11) angeordnet ist.

14. Beatmungsmaske nach einem der Ansprüche 1 bis 13, **dadurch gekennzeichnet, daß** im Bereich mindestens eines der Endsegmente (7, 8) eine Längenjustierung angeordnet ist.

15. Beatmungsmaske nach einem der Ansprüche 1 bis 14, **dadurch gekennzeichnet, daß** im Bereich des Nackenbandes (6) mindestens eine Längenjustierung angeordnet ist.

16. Beatmungsmaske nach einem der Anspruch 1 bis 15, **dadurch gekennzeichnet, daß** im Bereich des Nackenbandes (6) mindestens ein Verschluß angeordnet ist.

17. Beatmungsmaske nach Anspruch 16, **dadurch gekennzeichnet, daß** das Nackenband (6) zwei jeweils seitlich und einander gegenüberliegend angeordnete Verschlüsse aufweist.
